# EUROPÄISCHE PATENTSCHRIFT

(11) **EP 2 158 870 B1**
(45) Veröffentlichungstag und Bekanntmachung des Hinweises auf die Patenterteilung: **01.06.2011**
(21) Anmeldenummer: 08015004.8
(22) Anmeldetag: 26.08.2008
(51) Int. Cl.: A61C 1/05

(54) **Fluidbetriebener medizinischer, insbesondere dentaler, Handgriff**
Fluid-operated medical, in particular dental handle
Poignée hydraulique médicale, en particulier dentaire

(43) Veröffentlichungstag der Anmeldung: 03.03.2010
(73) Patentinhaber: W & H Dentalwerk Bürmoos GmbH, 5111 Bürmoos (AT)
(72) Erfinder: Rothenwänder, Michael, 5112 Lamprechtshausen (AT); Kment, Christoph, 1130 Wien (AT); Kölndorfer, Jürgen, 1100 Wien (AT); Brugger, Wilhelm, Dr., 5071 Wals-Siezenheim (AT)
(74) Vertreter: Benda, Ralf

(56) Entgegenhaltungen:
- US-A- 5 538 423
- US-A1- 2004 005 528
- US-A1- 2005 033 544

## Beschreibung

Die vorliegende Erfindung betrifft einen fluidbetriebenen medizinischen, insbesondere dentalen, Handgriff mit einer Vorrichtung zur Drehzahlbegrenzung nach dem Oberbegriff des Anspruchs 1 und ein Verfahren zum Begrenzen der Drehzahl eines derartigen Handgriffs.

Aus der Patentschrift US 3,578,872 ist ein derartiger fluidbetriebener Handgriff mit einer Vorrichtung zur Drehzahlbegrenzung bekannt. Am Handgriff ist dazu eine mit dem Laufrad umlaufende, U-förmige Aufnahme vorgesehen, in der sich ein elastischer O-Ring befindet. Am Außenrand der dem Laufrad abgewandten Stirnseite weist die U-förmige Aufnahme ein Vielzahl von Öffnungen auf. Der elastische Ring dehnt sich mit zunehmender Drehzahl infolge der Zentrifugalkraft aus und überdeckt dadurch die Öffnungen teilweise, wodurch der Durchsatz der Treibluft durch die Öffnungen und somit auch durch das Laufrad verringert wird. Die Begrenzung der Drehzahl des Handgriffs erfolgt also durch die Veränderung des wirksamen Querschnitts der Leitung des Antriebsfluids.

Einer der Nachteile dieser Vorrichtung zur Drehzahlbegrenzung liegt darin, dass der O-Ring aufgrund von Materialalterung und äußeren Einflüssen seine Elastizität verliert, wodurch die die Vorrichtung zur Drehzahlbegrenzung nur mehr eingeschränkt oder gar nicht mehr funktioniert.

Aus dem Stand der Technik ist des Weiteren eine Vielzahl von nicht gattungsgemäßen Handgriffen mit einer Drehzahlregelung bekannt, siehe zum Beispiel die Anmeldeschriften US 2007/0190484 A1 oder US 5538423. Diese Handgriffe umfassen einen Regelkreis mit einem Drehzahlsensor zur Ermittlung eines Drehzahl-Istwertes des Laufrads oder des Werkzeugs, einer Steuerung zum Vergleich des Drehzahl-Istwertes mit einem DrehzahlSollwert und mit einem Stellelement, oftmals einem Steuerventil, über das der Volumenstrom des Antriebsfluids verändert werden kann. Der Aufbau dieser Handgriffe mit dem Regelkreis ist aufwendig und kostspielig und häufig ist ihr Betriebsverhalten instabil.

Der vorliegenden Erfindung liegt daher die Aufgabe zugrunde einen fluidbetriebenen medizinischen, insbesondere dentalen, Handgriff mit einer Drehzahlbegrenzung zu schaffen, der die Nachteile der Vorrichtung zur Drehzahlbegrenzung aus der Patentschrift US 3,578,872 nicht aufweist.

Gemäß einem Ausführungsbeispiel wird dies durch einen medizinischen, insbesondere dentalen, fluidbetriebenen Handgriff erreicht, der ein durch ein Antriebsfluid in Drehung versetzbares Drehteil zum Antrieb eines mit dem Drehteil verbindbaren Werkzeugs und einen durch das Drehteil angetriebenen elektrodynamischen Wandler zur Induktion elektrischer Spannung aufweist. Der elektrodynamische Wandler umfasst zumindest eine Spule und ein Magnetelement. Im Handgriff ist des Weiteren zumindest ein Schaltelement zum wahlweisen Öffnen und Schließen eines Stromkreises zwischen den beiden Enden der zumindest einen Spule vorgesehen, so dass bei geschlossenem Stromkreis - durch die vom elektrodynamischen Wandler erzeugte Induktionsspannung - in der Spule des elektrodynamischen Wandlers elektrischer Strom und ein die Drehzahl des Drehteils und des damit verbindbaren Werkzeugs abbremsendes Induktionsmagnetfeld induzierbar sind.

Ein Vorteil des Handgriffs und insbesondere der Vorrichtung zur Drehzahlbegrenzung, die den elektrodynamischen Wandler und das Schaltelement umfasst, liegt darin, dass zur Drehzahlbegrenzung und insbesondere zur Erzeugung der Bremskraft zum Abbremsen des Drehteils keine mechanischen, verschleissanfälligen Bauteile benötigt werden. Die Bremswirkung wird durch elektrodynamische Effekte erzielt, insbesondere durch Induktion, wobei durch die Drehung des Rotors des elektrodynamischen Wandlers eine Spannung in der Spule des elektrodynamischen Wandlers induziert wird. Werden die beiden Enden der Spule durch das Schaltelement miteinander verbunden, so fließt elektrischer Strom durch diese Spule, wobei dieser induzierte Strom die Bildung eines im Folgenden als Induktionsmagnetfeld bezeichneten Magnetfelds hervorruft. Dieses Induktionsmagnetfeld wirkt der Induktionsursache, das heißt dem Magnetfeld des Magnetelements des elektrodynamischen Wandlers, entgegen und bremst somit den Rotor des elektrodynamischen Wandlers und das damit verbundene Drehteil und Werkzeug des Handgriffs ab. Die Bremswirkung beruht auf dem Prinzip der dem Fachmann bekannten Lenz'schen Regel, so dass darauf nicht weiter eingegangen wird.

Die Drehzahlbegrenzung begrenzt die Drehzahl des Drehteils und des Werkzeugs auf einen Maximalwert, insbesondere bei schwacher Belastung oder bei Leerlauf des Handgriffs, um damit die Lärmemission des Handgriffs zu verringern und die mechanische Belastung der im Handgriff befindlichen, das Drehteil lagernden Kugellager zu verringern. Auch ermöglicht die Drehzahlbegrenzung ein sanfteres Aufsetzen des Werkzeugs auf die Behandlungsstelle. Gemäß einem Ausführungsbeispiel begrenzt das Schaltelement die Drehzahl des Drehteils und des damit verbindbaren Werkzeugs auf einen Wert im Bereich von etwa 300.000 - 150.000 Umdrehungen pro Minute, bevorzugt auf einen Wert im Bereich von etwa 275.000 - 200.000 Umdrehungen pro Minute, besonders bevorzugt auf in etwa 250.000 Umdrehungen pro Minute.

Durch die Auswahl von Schaltelementen mit unterschiedlichen Schaltwerten ist es des Weiteren möglich, in Handgriffen Drehzahlbegrenzungen mit unterschiedlichen maximalen Drehzahlen zu implementieren.

Der elektrodynamische Wandler, im Folgenden auch als Generator bezeichnet, weist einen Stator und einen Rotor auf, wobei der Rotor mit dem Drehteil verbunden ist oder als Teil des Drehteils ausgebildet ist. Das Drehteil umfasst zum Beispiel das durch das Antriebsfluid beaufschlagte Laufrad des Handgriffs, eine im Handgriff drehbar gelagerte Welle, zum Beispiel eine Hohlwelle zur lösbaren Aufnahme des Behandlungswerkzeugs, oder den Schaft eines im Handgriff aufnehmbaren Werkzeugs. Bevorzugt umfasst der Rotor das Magnetelement und der Stator die zumindest eine Spule, selbstverständlich ist jedoch auch eine umgekehrte Ausgestaltung möglich. Besonders bevorzugt ist das Magnetelement als Permanentmagnet ausgebildet, der insbesondere als Scheibenmagnet am Drehteil befestigt ist. Alternativ ist das Drehteil selbst magnetisch, zum Beispiel aufgrund seiner Herstellung aus magnetischem Material oder durch Aufmagnetisierung.

Das Schaltelement oder Teile davon verbinden die beiden Enden der zumindest einen, bevorzugt aus Kupferdraht hergestellten, Spule des Generators und /oder schließen diese kurz. Zumindest Teile des Schaltelements sind somit Teil eines Stromkreises, der neben der Spule des Generators gegebenenfalls auch noch andere elektrische oder elektronische Bauteil umfasst, zum Beispiel elektrisch leitende Drähte oder eine elektrische Last.

Das Schaltelement schließt den Stromkreis bei Erreichen oder Überschreiten eines Schaltwerts oder eines Schaltbereichs. Als Auslöser des Schalters dient die vom elektrodynamischen Wandler induzierte Spannung und / oder ein davon abhängiger Parameter, wobei die induzierte Spannung direkt proportional zur Drehzahl des Drehteils oder des Werkzeugs ist. Erreicht zum Beispiel die induzierte Spannung den vorbestimmten Schaltwert (die so genannte Schaltspannung oder Durchlassspannung) einer Diode, die als Schaltelement dient, so schaltet diese durch, d.h. sie wird in einen niederohmigen Zustand versetzt, wodurch ein hoher elektrischer Stromfluss durch die Diode und somit durch die Spule des elektrodynamischen Wandlers möglich wird. Umfasst das Schaltelement zum Beispiel einen elektrischen Widerstand, so setzt der Stromfluss im Vergleich zur Diode weniger abrupt ein, so dass hier nach Überschreiten eines Schaltwerts ein Schaltbereich vorliegt, in dem, in Abhängigkeit von der induzierten Spannung des Generators, der elektrische Stromfluss ansteigt. Alternativ ist es auch möglich, dass der Schaltbereich durch mehrere Schaltwerte gebildet ist. Dies wird zum Beispiel dadurch erreicht, dass das Schaltelement mehrere Schalteinheiten oder Schalter umfasst, die der Reihe nach mit steigender induzierter Spannung aktivierbar sind.

Gemäß einem Ausführungsbeispiel ist die zumindest eine Spule um einen weichmagnetischen Spulenkern gewickelt, der den magnetischen Fluss des Magnetelements des Generators bündelt und zur Spule lenkt. Insbesondere ist der aus einer oder mehreren, bevorzugt elektrisch voneinander isolierten, Schichten bestehende weichmagnetische Spulenkern ringförmig geformt und umgibt das Magnetelement an seinem Außenumfang. Damit wird insbesondere wenn der elektrodynamische Wandler mehrere Spulen umfasst, deren Einbau im Handgriff erleichtert.

Durch das Vorsehen mehrerer, bevorzugt dreier, Spulen wird eine erhöhte Bremswirkung erzielt. Sind die Spulen besonders bevorzugt im Wesentlichen gleichmäßig um das Magnetelement angeordnet, so wird die Laufruhe des Drehteils und des Werkzeugs beim Abbremsen nur wenig oder gar nicht beeinträchtigt. Gemäß einem bevorzugten Ausführungsbeispiel ist jede Spule mit einem Schaltelement verbunden, wobei die Schaltelemente gleiche oder ähnliche Schaltwerte aufweisen, so dass eine im Wesentlichen simultane Induktion der Induktionsmagnetfelder möglich ist. Durch die gleichen oder ähnlichen Schaltwerte wird das gleichmäßige Abbremsen des Drehteils und des Werkzeugs noch zusätzlich unterstützt. Alternativ ist jede Spule mit einem Schaltelement verbunden, wobei die Schaltelemente unterschiedliche Schaltwerte aufweisen, so dass eine sequentielle Induktion der Induktionsmagnetfelder realisierbar ist.

Das Schaltelement kann jeden bekannten Schalter, insbesondere jeden elektrischen oder elektronischen Schalter umfassen. Gemäß einem Ausführungsbeispiel umfasst das Schaltelement eine oder mehrere Halbleiterdioden, insbesondere Thyristoren, zum Beispiel zwei antiseriell angeordnete Zener-Dioden, eine Zweirichtungs-Diode (Diac) oder eine elektromagnetische Strahlung emittierende Leuchtdiode und ein die Strahlung empfangendes Halbleiterelement, zum Beispiel eine Photodiode, einen Phototransistor oder einen Photo-Triac. Der Vorteil der Verwendung von Halbleiterdioden besteht unter anderem darin, dass außer einer oder zwei Dioden keine weiteren Bauteile für die Schaltfunktion benötigt werden.

Gemäß einem alternativen Ausführungsbeispiel umfasst das Schaltelement zumindest einen elektrischen Widerstand, insbesondere einen spannungsabhängigen Widerstand oder einen temperaturabhängigen Widerstand. Der Vorteil der Verwendung von Widerständen liegt in ihrer im Vergleich zu Halbleiterdioden höheren Robustheit und Beständigkeit gegenüber äußeren Einflüssen, zum Beispiel erhöhten Temperaturen.

Gemäß einem weiteren Ausführungsbeispiel weist der elektrodynamische Wandler zumindest eine Steuerspule auf, in der ebenfalls eine elektrische Spannung und elektrischer Strom induziert werden. Die Steuerspule ist jedoch nicht wie die übrigen Spulen, die zur Unterscheidung als Bremsspulen bezeichnet werden und die das das Drehteil abbremsende Induktionsmagnetfeld erzeugen, mit einem Schaltelement versehen, sondern sie ist mit einem elektrischer Verbraucher oder operativ mit einem Sensor verbunden. Der Verbraucher, zum Beispiel eine Leuchtdiode oder ein Heizelement, sind Teil des Schaltelements oder operativ mit diesem verbunden, um die Schaltfunktion auszulösen.

Der Sensor ist zum Beispiel dazu vorgesehen, einen Parameter der in der Steuerspule induzierten Spannung oder des zwischen den beiden Enden der Steuerspule fließenden induzierten Strom zu messen, insbesondere die Frequenz der induzierten Wechselspannung, und ein dem Messwert entsprechendes Signal an eine Steuereinheit mit einem elektronischen Schaltkreis oder einen Mikrokontroller zu senden. Die Steuereinheit oder der Mikrokontroller sind mit dem zumindest einen Schaltelement der Bremsspule verbunden oder sind Teil des Schaltelements und öffnen oder schließen den die Enden der Bremsspule verbindenden Stromkreis auf Basis des Sensorsignals durch Aktivieren oder Deaktivieren des Schaltelements. Das Schaltelement kann dabei als elektrisches, elektronisches oder auch als mechanisches Schaltelement ausgebildet sein.

Gemäß einem anderen Ausführungsbeispiel sind der elektrodynamische Wandler und das gesamte Schaltelement im Handgriff aufgenommen, wodurch die Handhabbarkeit des Handgriffs erheblich verbessert wird.

Ein Verfahren zur Drehzahlbegrenzung des oben beschriebenen, medizinischen, insbesondere dentalen, fluidbetriebenen Handgriffs ist dadurch gekennzeichnet dass, durch das in Drehung versetzen des Drehteils in der zumindest einen Spule des elektrodynamischen Wandlers eine Spannung induziert wird und dass das Schaltelement bei Erreichen oder Überschreiten des Schaltwerts des Schaltelements einen Stromkreis zwischen den beiden Enden der zumindest einen Spule schließt, so dass in der Spule des elektrodynamischen Wandlers ein elektrischer Strom und ein die Drehzahl des Drehteils und des damit verbindbaren Werkzeugs abbremsendes Induktionsmagnetfeld induziert werden.

Die Erfindung wird nachfolgend anhand bevorzugter Ausführungsbeispiele und Bezug nehmend auf die beigefügten Zeichnungen erläutert:
Figur 1 zeigt ein Ausführungsbeispiel eines medizinischen, insbesondere dentalen, fluidbetriebenen Handgriffs mit einer Vorrichtung zur Drehzahlbegrenzung.
Figur 2 zeigt in einer Schnittdarstellung den Kopfteil des Handgriffs der Figur 1 mit der einen elektrodynamischen Wandler und ein damit verbundenes Schaltelement umfassenden Vorrichtung zur Drehzahlbegrenzung.
Figur 3 zeigt in einer schematischen Darstellung ein Ausführungsbeispiel einer Vorrichtung zur Drehzahlbegrenzung, wobei der elektrodynamische Wandler drei Induktionsspulen aufweist.
Die Figuren 4A - 4D zeigen unterschiedliche Ausführungsbeispiele des Schaltelements der Vorrichtung zur Drehzahlbegrenzung.
Figur 5 zeigt in einer schematischen Darstellung ein Ausführungsbeispiel einer Vorrichtung zur Drehzahlbegrenzung, wobei der elektrodynamische Wandler eine Bremsspule zur Erzeugung eines das Drehteil abbremsenden Induktionsmagnetfelds und eine Steuerspule aufweist.
Figur 6 zeigt ein alternatives Ausführungsbeispiel eines Kopfteils eines medizinischen, insbesondere dentalen, fluidbetriebenen Handgriffs mit einer Vorrichtung zur Drehzahlbegrenzung, die ein scheibenförmiges, die Werkzeugaufnahme umgebendes Magnetelement aufweist.

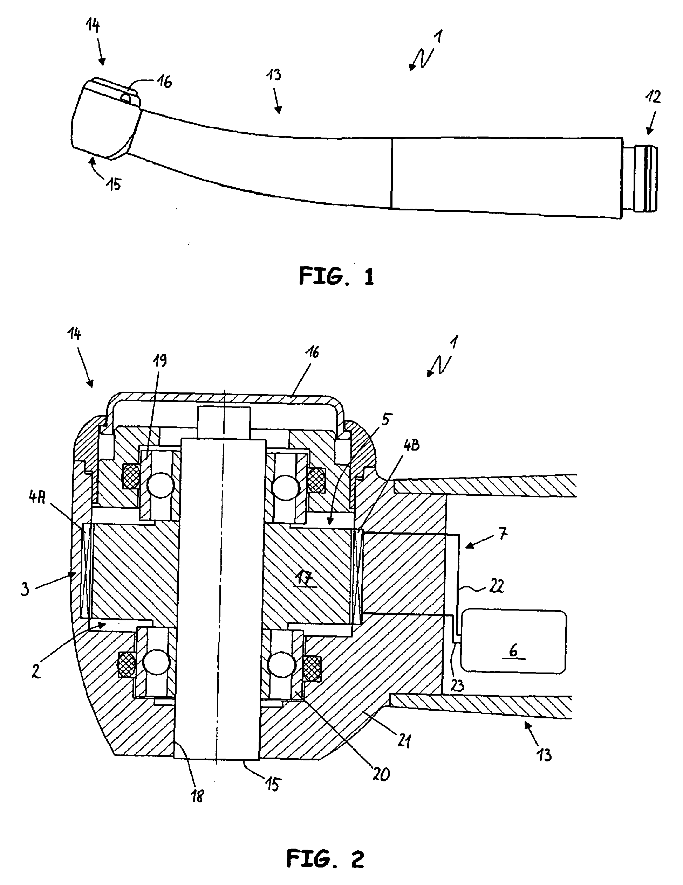

Der in der Figur 1 dargestellte medizinische, insbesondere dentale, fluidbetriebene Handgriff 1 ist als längliches, rohrförmiges Instrument ausgebildet, das an seinem ersten Ende einen Anschluss 12 an eine Fluidquelle, insbesondere eine Druckluftquelle, aufweist. Der Handgriff 1 umfasst einen gebogenen oder zwei gewinkelt zueinander angeordnete Abschnitte aufweisenden Griffteil 13 und einen daran anschließenden Kopfteil 14. Am Kopfteil 14 ist eine Werkzeugöffnung 15 vorgesehen, durch die ein Werkzeug lösbar in den Kopfteil 14 einsteckbar ist. Die Werkzeugöffnung 15 ist seitlich am Kopfteil 14 angeordnet, so dass das Werkzeug gewinkelt zum Griffteil 13 oder dessen Längsachse aus dem Kopfteil 14 ragt. An dem der Werkzeugöffnung 15 gegenüberliegenden Ende des Kopfteils 14 ist ein Drucktaster 16 vorgesehen, der mit einer im Kopfteil 14 angeordneten Werkzeuglösevorrichtung zusammenwirkt, um das Werkzeug aus dem Kopfteil 14 frei zu geben. Selbstverständlich kann der Handgriff 1 auch andere bekannte Formen aufweisen, zum Beispiel pistolenförmig oder gerade ausgebildet sein.

Von der Anschlussvorrichtung 12 erstreckt sich zumindest eine Fluidleitung für das Antriebsfluid in Richtung des Kopfteils 14. Gegebenenfalls erstrecken sich weitere Medienleitungen, zum Beispiel eine oder mehrere Medienleitungen für Kühlmedien, Lichtleiter oder elektrische Leitungen von der Anschlussvorrichtung 12 ausgehend durch den Handgriff 1. Die Fluidleitung fördert das Antriebsfluid, zum Beispiel Druckluft oder Wasser, zu einer Antriebseinheit, die zumindest ein in Drehung versetzbares Drehteil 2 umfasst, zum Beispiel ein Laufrad, einen Rotor eines Luft- oder Lamellenmotors, eine Welle, eine Spannvorrichtung für das Werkzeug etc.

Gemäß der Ausführungsform des Handgriffs 1 nach Figur 2 umfasst das Drehteil 2 das Laufrad 17 und eine Hohlwelle 18, auf der das Laufrad 17 befestigt ist und in der die Spannvorrichtung für das Werkzeug angeordnet ist oder die Teil der Spannvorrichtung ist. Das Drehteil 2 und das damit verbindbare Werkzeug sind durch zwei Wälzlager 19, 20 drehbar im Handgriff 1 gelagert. Die Wälzlager 19, 20 sind an drehfesten Bauteilen des Handgriffs 1, zum Beispiel der Außenhülse 21 , gelagert.

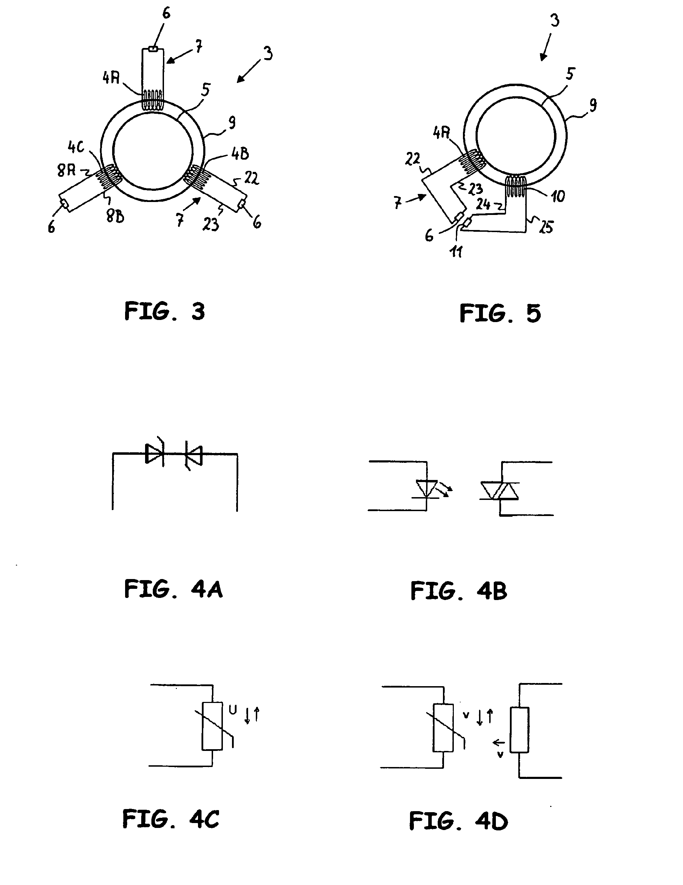

Im Kopfteil 14 des Handgriffs 1 ist ein elektrodynamischer Wandler oder Generator 3 vorgesehen, der einen Rotor und einen Stator umfasst. Der Stator wird durch zumindest eine, bevorzugt mehrere, Spulen 4A, 4B, 4C und einen weichmagnetischen Spulenkern 9 (Figur 3), um den die Spulen 4A, 4B, 4C gewickelt sind, gebildet. Die Spulen 4A, 4B, 4C und der Spulenkern 9 umgeben den ein Magnetelement 5 aufweisenden Rotor. Der Rotor umfasst gemäß Figur 2 das zumindest teilweise magnetische Laufrad 17, wobei bevorzugt die Flügel des Laufrads 17 durch Aufmagnetisierung magnetisch sind.

Durch Beaufschlagen des Laufrads 17 mit dem Antriebsfluid und durch das in Drehung versetzen desselben wird somit auch der elektrodynamische Wandler 3 aktiviert und in den Spulen 4A, 4B, 4C eine Spannung induziert. Die Höhe der in den Spulen 4A, 4B, 4C induzierten Spannung ist dabei direkt proportional zur Drehzahl des Rotors, des Drehteils 2 und des Werkzeugs. Die Spulen 4A, 4B, 4C sind über ihre Enden 8A, 8B (siehe Figur 3) und elektrische Leitungen 22, 23 mit den Schaltelementen 6 verbunden.

Die Schaltelemente 6 sind zwischen einem ersten, offenen Zustand, in dem die Verbindung zwischen den Enden 8A, 8B der Spulen 4A, 4B, 4C unterbrochen ist, und einem zweiten, geschlossenen Zustand, in dem die Enden 8A, 8B miteinander verbunden sind, schaltbar. In diesem zweiten Zustand fließt aufgrund der induzierten Spannung elektrischer Strom durch den von den Spulen 4A, 4B, 4C, den Leitungen 22, 23 und dem Schaltelement 6 gebildeten Stromkreis 7. Das Umschalten vom ersten, offenen Zustand, der der Grundzustand des Schaltelements 6 ist, in den geschlossenen Zustand erfolgt in Abhängigkeit der Höhe der induzierten Spannung. Somit fließt elektrischer Strom erst dann durch den Stromkreis 7 und insbesondere die Spulen 4A, 4B, 4C, wenn die Drehzahl des Drehteils 2 hoch genug ist, um eine Spannung zu induzieren, die den Schaltwert des Schaltelements 6 erreicht oder überschreitet. Sinkt die induzierte Spannung aufgrund der geringen Drehzahl des Drehteils 2 unter den Schaltwert, so kehrt das Schaltelement 6 selbsttätig in seinen Grundzustand zurück.

Wie bereits weiter oben erläutert, löst der durch die Spulen 4A, 4B, 4C fließende induzierte Strom die Bildung eines Induktionsmagnetfelds in den Spulen 4A, 4B, 4C aus. Dieses Induktionsmagnetfeld wirkt der Induktionsursache, das heißt dem Magnetfeld des Magnetelements 5 des elektrodynamischen Wandlers 3 entgegen und bremst somit den Rotor des elektrodynamischen Wandlers 3, das Drehteil 2 und das Werkzeug des Handgriffs 1 ab. Da mit steigender Drehzahl auch die induzierte Spannung, der induzierte Strom und die Stärke des Induktionsmagnetfelds zunehmen, wird über diese Vorrichtung zur Drehzahlbegrenzung eine zuverlässige Begrenzung der Drehzahl des Drehteils 2 und des Werkzeugs auf eine maximale Drehzahl erreicht.

Die Figur 3 zeigt in einer Aufsicht den elektrodynamischen Wandler 3 und drei Stromkreise 7, deren Spulen 4A, 4B, 4C im Wesentlichen regelmäßig um das Magnetelement 5 angeordnet sind. Jeder Spule 4A, 4B, 4C und jedem Stromkreis 7 ist ein Schaltelement 6 zugeordnet, wobei bevorzugt die drei Schaltelemente 6 im Wesentlichen gleiche Schaltwerte aufweisen, so dass die drei Schaltelemente 6 die Stromkreise 7 mit steigender Drehzahl im Wesentlichen simultan schließen bzw. mit sinkender Drehzahl simultan öffnen und dem gemäß die Induktionsmagnetfelder ebenfalls im Wesentlichen gleichzeitig induziert werden bzw. zusammenbrechen.

In den Figuren 4A und 4C sind zwei mögliche Ausführungsformen der Schaltelemente 6 abgebildet, wie sie zum Beispiel in den Stromkreisen 7 der Figur 3 verwendet werden können. Die Figur 4A zeigt zwei antiseriell geschaltete Zener Dioden, die bei Überschreiten ihrer Schaltspannung niederohmig werden, so dass ein hoher elektrischer Stromfluss durch den Stromkreis 7 und die Spulen 4A, 4B, 4C fließt. Die Figur 4C zeigt einen spannungsabhängigen Widerstand, dessen Widerstandswert mit steigender induzierter Spannung abnimmt, so dass in Folge ein hoher elektrischer Stromfluss durch den Stromkreis 7 und die Spulen 4A, 4B, 4C fließt.

Die Figur 5 zeigt eine alternative Ausführungsform einer Vorrichtung zur Drehzahlbegrenzung, die wiederum einen elektrodynamischen Wandler 3 und einen Stromkreis 7 mit einer Spule 4A, den Leitern 22, 23 und einem Schaltelement 6 umfasst. Diese Bauteile und die mit den gleichen Bezugszeichen versehenen Bauteile der Figur 3 weisen denselben Aufbau und dieselbe Funktionsweise auf. Zusätzlich weist die Vorrichtung zur Drehzahlbegrenzung der Figur 5 eine Steuerspule 10 auf, die über elektrische Leitungen 24, 25 mit einem elektrischen Verbraucher 11 verbunden ist. Der elektrische Verbraucher 11 ist dem Schaltelement 6 zugeordnet oder gegenüber dem Schaltelement 6 angeordnet.

Bei Betrieb des elektrodynamischen Wandlers 3 wird in der Steuerspule 10 eine Spannung induziert, die einen Stromfluss bewirkt, über den der Verbraucher 11 betrieben wird. Der Verbraucher 11 kann zum Beispiel eine oder mehrere elektromagnetische Strahlung emittierende Leuchtdioden umfassen (siehe Figur 4B), die, wenn der Wert der in der Steuerspule 10 induzierten Spannung hoch genug ist, um ihren Schaltwert zu überschreiten, Strahlung in Richtung des Schaltelements 6 emittiert. Das Schaltelement 6 wird durch ein die Strahlung empfangendes Halbleiterelement, zum Beispiel eine Photodiode gebildet, das, sobald es von der Leuchtdiode emittierte Strahlung empfängt, in den geschlossenen Zustand wechselt, wodurch der Stromkreis 7 schließt und in der Spule 4A wie oben beschrieben das das Drehteil 2 bremsende Induktionsmagnetfeld aufgebaut wird.

Die Steuerspule 10 und die Bremsspule 4A sind entweder nebeneinander angeordnet, so wie dies in Figur 5 dargestellt ist, oder die beiden Spulen sind zwecks Platzersparnis übereinander gewickelt, insbesondere ist die Steuerspule 10 über die Bremsspule 4A gewickelt. Gemäß einer bevorzugten Ausführungsform ist die Steuerspule 10 auch noch mit einem Gleichrichter verbunden, um den induzierten Wechselstrom in Gleichstrom zu wandeln.

In der Figur 4D ist der elektrische Verbraucher 11 durch einen oder mehrere Wärme emittierende Heizelemente gebildet, zum Beispiel durch eine Heizfolie. Durch den in den Leitungen 24 und 25 fließenden Strom wird das Heizelement aufgeheizt und strahlt Wärmestrahlung in Richtung des Schaltelements 6 ab, das zum Beispiel einen temperaturabhängigen Widerstand (NTC-Widerstand) umfasst. Mit zunehmender Temperatur sinkt der Widerstandswert des temperaturabhängigen Widerstands, wodurch der Stromkreis 7 schließt, Strom durch den Stromkreis 7 und die Spule 4A fließt und in der Spule 4A wiederum das das Drehteil 2 bremsende Induktionsmagnetfeld aufgebaut wird.

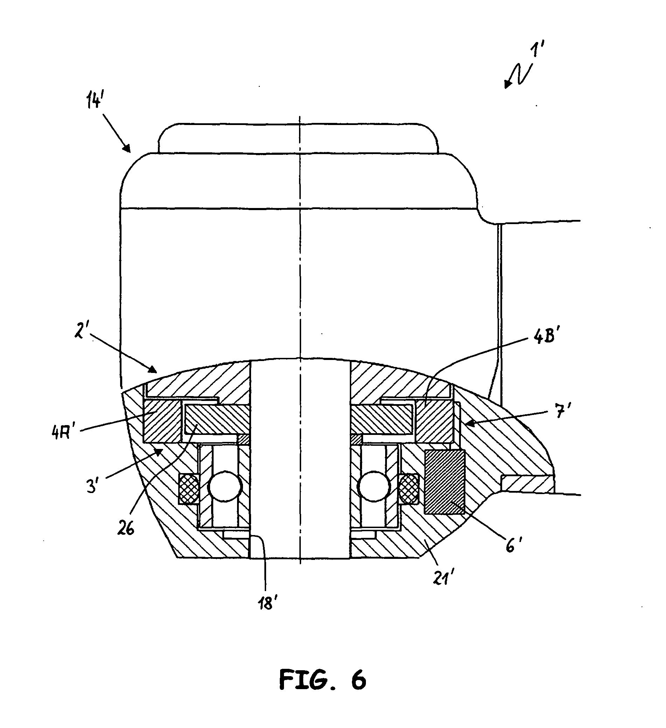

Der in der Figur 6 dargestellte Kopfteil 14' des Handgriffs 1' gleicht in seinem Aufbau dem in Figur 2 dargestellten Kopfteil 14. Der Unterschied zu dem Kopfteil 14 besteht darin, dass das Magnetelement des elektrodynamische Wandlers 3' als ein scheibenförmiger Permanentmagneten 26 ausgebildet ist, zum Beispiel als ein Neodym-Eisen-Bor-Magnet, der an dem Drehteil 2', insbesondere an der Welle 18', angebracht ist. Die das Magnetelement 26 umgebenden Spulen 4A', 4B' sind auf Schultern des Kopfgehäuses 21' gelagert. Jede Spule 4A', 4B' ist zur Bildung eines Stromkreises 7' mit einem Schaltelement 6' (nur eines ist dargestellt) über elektrische Leitungen verbunden. Im Gegensatz zum Handgriff 1 der Figur 2, bei dem zumindest ein, bevorzugt alle, Schaltelement 6 im Griffteil 13 vorgesehen sind, sind die Schaltelemente 6' und der gesamte Stromkreis 7' des Handgriffs 1' im Kopfteil 14' angeordnet.

## Patentansprüche

1. Medizinischer, insbesondere dentaler, fluidbetriebener Handgriff (1, 1') mit einem durch ein Antriebsfluid in Drehung versetzbaren Drehteil (2, 2') zum Antrieb eines mit dem Drehteil (2, 2') verbindbaren Werkzeugs und mit einem durch das Drehteil (2, 2') angetriebenen elektrodynamischen Wandler (3, 3') zur Induktion elektrischer Spannung, wobei der elektrodynamische Wandler (3, 3') zumindest eine Spule (4A, 4B, 4C; 4A', 4B') und ein Magnetelement (5, 26) umfasst, **gekennzeichnet durch** zumindest ein Schaltelement (6, 6') zum wahlweisen Öffnen und Schließen eines Stromkreises (7, 7') zwischen den beiden Enden (8A, 8B) der zumindest einen Spule (4A, 4B, 4C; 4A', 4B'), so dass bei geschlossenem Stromkreis (7, 7') in der Spule (4A, 4B, 4C; 4A', 4B') des elektrodynamischen Wandlers (3, 3') ein elektrischer Strom und ein die Drehzahl des Drehteils (2, 2') und des damit verbindbaren Werkzeugs abbremsendes Induktionsmagnetfeld induzierbar sind.

2. Medizinischer, insbesondere dentaler, fluidbetriebener Handgriff (1, 1'), nach Anspruch 1 **dadurch gekennzeichnet, dass**
das Magnetelement (5, 26) am Drehteil (2, 2') vorgesehen ist, so dass das Magnetelement (5, 26) durch das Drehteil (2, 2') in Drehung versetzbar ist.

3. Medizinischer, insbesondere dentaler, fluidbetriebener Handgriff (1, 1'), nach Anspruch 1 oder 2 **dadurch gekennzeichnet, dass**
die zumindest eine Spule (4A, 4B, 4C; 4A', 4B') um einen weichmagnetischen Spulenkern (9) gewickelt ist.

4. Medizinischer, insbesondere dentaler, fluidbetriebener Handgriff (1, 1'), nach Anspruch 3 **dadurch gekennzeichnet, dass**
der aus einer oder mehreren, bevorzugt elektrisch voneinander isolierten, Schichten bestehende weichmagnetische Spulenkern (9) ringförmig geformt ist und das Magnetelement (5, 26) an seinem Außenumfang umgibt.

5. Medizinischer, insbesondere dentaler, fluidbetriebener Handgriff (1, 1'), nach einem der vorhergehenden Ansprüche, **dadurch gekennzeichnet, dass**
der elektrodynamische Wandler (3, 3') mehrere, bevorzugt drei, Spulen (4A, 4B, 4C; 4A', 4B') umfasst, die besonders bevorzugt im Wesentlichen gleichmäßig um das Magnetelement (5, 26) angeordnet sind.

6. Medizinischer, insbesondere dentaler, fluidbetriebener Handgriff (1, 1'), nach einem der vorhergehenden Ansprüche, **dadurch gekennzeichnet, dass**
das Schaltelement (6, 6') eine oder mehrere Halbleiterdioden, insbesondere Thyristoren, umfasst.

7. Medizinischer, insbesondere dentaler, fluidbetriebener Handgriff (1, 1'), nach Anspruch 6 **dadurch gekennzeichnet, dass**
das Schaltelement (6, 6') zwei antiseriell angeordnete Zener-Dioden oder eine Zweirichtungs-Diode umfasst.

8. Medizinischer, insbesondere dentaler, fluidbetriebener Handgriff (1, 1'), nach Anspruch 6 **dadurch gekennzeichnet, dass**
das Schaltelement (6, 6') zumindest eine elektromagnetische Strahlung emittierende Leuchtdiode und zumindest ein die Strahlung empfangendes Halbleiterelement aufweist.

9. Medizinischer, insbesondere dentaler, fluidbetriebener Handgriff (1, 1'), nach einem der vorhergehenden Ansprüche, **dadurch gekennzeichnet, dass**
das Schaltelement (6, 6') zumindest einen elektrischen Widerstand, insbesondere einen spannungsabhängigen Widerstand oder einen temperaturabhängigen Widerstand, umfasst.

10. Medizinischer, insbesondere dentaler, fluidbetriebener Handgriff (1, 1'), nach einem der vorhergehenden Ansprüche, **dadurch gekennzeichnet, dass**
der elektrodynamische Wandler (3, 3') zumindest eine Steuerspule (10) umfasst, an die ein elektrischer Verbraucher (11) angeschlossen ist oder die operativ mit einem Sensor verbunden ist.

11. Medizinischer, insbesondere dentaler, fluidbetriebener Handgriff (1, 1'), nach einem der vorhergehenden Ansprüche, **dadurch gekennzeichnet, dass**
das Schaltelement (6, 6') einen elektronischen Schaltkreis, insbesondere einen Mikrokontroller, umfasst.

12. Medizinischer, insbesondere dentaler, fluidbetriebener Handgriff (1, 1'), nach einem der vorhergehenden Ansprüche, **dadurch gekennzeichnet, dass**
das Schaltelement (6, 6') die Drehzahl des Drehteils (2, 2') und des damit verbindbaren Werkzeugs auf einen Wert im Bereich von etwa 300.000 - 150.000 Umdrehungen pro Minute, bevorzugt auf einen Wert im Bereich von etwa 275.000 - 200.000 Umdrehungen pro Minute, besonders bevorzugt auf in etwa 250.000 Umdrehungen pro Minute begrenzt.

13. Medizinischer, insbesondere dentaler, fluidbetriebener Handgriff (1, 1'), nach einem der vorhergehenden Ansprüche, **dadurch gekennzeichnet, dass**
der elektrodynamische Wandler (3, 3') und das gesamte Schaltelement (6, 6') im Handgriff (1, 1') aufgenommen sind.

14. Medizinischer, insbesondere dentaler, fluidbetriebener Handgriff (1, 1'), nach einem der vorhergehenden Ansprüche, **dadurch gekennzeichnet, dass**
mehrere Spulen (4A, 4B, 4C; 4A', 4B') vorgesehen sind, wobei jede Spule (4A, 4B, 4C; 4A', 4B') mit einem Schaltelement (6, 6') verbunden ist und die Schaltelemente (6, 6') unterschiedliche Schaltwerte aufweisen, so dass die Induktionsmagnetfelder sequentielle induzierbar sind.

15. Verfahren zur Drehzahlbegrenzung eines medizinischen, insbesondere dentalen, fluidbetriebenen Handgriffs (1, 1') nach einem der vorhergehenden Ansprüche, **dadurch gekennzeichnet dass**,
durch das in Drehung versetzen des Drehteils (2, 2') in der zumindest einen Spule (4A, 4B, 4C; 4A', 4B') des elektrodynamischen Wandlers (3, 3') eine Spannung induziert wird und dass das Schaltelement (6, 6') einen Stromkreis (7, 7') zwischen den beiden Enden (8A, 8B) der zumindest einen Spule (4A, 4B, 4C; 4A', 4B') schließt, so dass in der Spule (4A, 4B, 4C; 4A', 4B') des elektrodynamischen Wandlers (3, 3') elektrischer Strom und ein die Drehzahl des Drehteils (2, 2') und des damit verbindbaren Werkzeugs abbremsendes Induktionsmagnetfeld induziert werden.

## Claims

1. A fluid-operated medical, inparticular dental, handle (1, 1') with a rotating part (2, 2') that can be induced to rotate by a driving fluid for driving a tool connectable to the rotating part (2, 2') and with an electrodynamic transducer (3, 3') driven by the rotating part (2, 2') for induction of an electric voltage, wherein the electrodynamic transducer (3, 3') comprises at least one coil (4A, 4B, 4C; 4A', 4B') and one magnetic element (5, 26), **characterized by**
at least one switch element (6, 6') for selective opening and closing of an electric circuit (7, 7') between the two ends (8A, 8B) of the at least one coil (4A, 4B, 4C; 4A', 4B'), so that with the electric circuit (7, 7') closed, an electric current and an induced magnetic field for decelerating the rotational speed of the rotating part (2, 2') and of the tool connectable thereto can be induced in the coil (4A, 4B, 4C; 4A', 4B') of the electrodynamic transducer (3, 3').

2. The fluid-operated medical, inparticular dental, handle (1, 1') according to claim 1, **characterized in that**
the magnetic element (5, 26) is provided on the rotating part (2, 2'), so that the magnetic element (5, 26) can be induced to rotation by the rotating part (2, 2').

3. The fluid-operated medical, inparticular dental, handle (1, 1') according to claim 1 or 2, **characterized in that**
the at least one coil (4A, 4B, 4C; 4A', 4B') is wound around a soft magnetic coil core (9).

4. The fluid-operated medical, inparticular dental, handle (1, 1') according to claim 3, **characterized in that**
the soft magnetic coil core (9) comprising one or more layers, which preferably are electrically insulated from each other, is shaped in a ring and surrounds the magnetic element (5, 26) on its outside circumference.

5. The fluid-operated medical, inparticular dental, handle (1, 1') according to one of the preceding claims, **characterized in that**
the electrodynamic transducer (3, 3') comprises multiple, preferably three, coils (4A, 4B, 4C; 4A', 4B'), which especially preferably are arranged substantially uniformly about the magnetic element (5, 26).

6. The fluid-operated medical, inparticular dental, handle (1, 1') according to one of the preceding claims, **characterized in that**
the switch element (6, 6') comprises one or more semiconductor diodes, in particular thyristors.

7. The fluid-operated medical, inparticular dental, handle (1, 1') according to claim 6, **characterized in that**
the switch element (6, 6') comprises two Zener diodes arranged in an antiserial configuration or a bidirectional diode.

8. The fluid-operated medical, inparticular dental, handle (1, 1') according to claim 6, **characterized in that**
the switch element (6, 6') comprises at least one electromagnetic radiation emitting illuminating diode and at least one semiconductor element receiving the radiation.

9. The fluid-operated medical, inparticular dental, handle (1, 1') according to one of the preceding claims, **characterized in that**
the switch element (6, 6') comprises at lease one electric resistor, in particular a voltage-dependent resistor or a temperature-dependent resistor.

10. The fluid-operated medical, inparticular dental, handle (1, 1') according to one of the preceding claims, **characterized in that**
the electrodynamic transducer (3, 3') comprises at least one control coil (10), to which an electric consumer (11) is connected or which is operatively connected to a sensor.

11. The fluid-operated medical, inparticular dental, handle (1, 1') according to one of the preceding claims, **characterized in that**
the switch element (6, 6') comprises an electronic circuit, in particular a microcontroller.

12. The fluid-operated medical, inparticular dental, handle (1, 1') according to one of the preceding claims, **characterized in that**
the switch element (6, 6') limits the rotational speed of the rotating part (2, 2') and of the tool connectable thereto to a value in the range of approximately 300.000 - 150.000 revolutions per minute, preferably to a value in the range from approximately 275.000 - 200.000 revolutions per minute, especially preferably to approximately 250,000 revolutions per minute.

13. The fluid-operated medical, inparticular dental, handle (1, 1') according to one of the preceding claims, **characterized in that**
the electrodynamic transducer (3, 3') and the entire switch element (6, 6') are accommodated in the handle (1, 1').

14. The fluid-operated medical, inparticular dental, handle (1, 1') according to one of the preceding claims, **characterized in that**
multiple coils (4A, 4B, 4C; 4A', 4B') are provided, wherein each coil (4A, 4B, 4C; 4A', 4B') is connected to a switch element (6, 6') and the switch elements (6, 6') are provided with different switching values, so that the induced magnetic fields are sequentially inducible.

15. A method for limiting the rotational speed of a fluid-operated medical, inparticular dental, handle (1, 1') according to one of the preceding claims, **characterized in that** a voltage is induced in the at least one coil (4A, 4B, 4C; 4A', 4B') of the electrodynamic transducer (3, 3') by initiating rotation of the rotating part (2, 2') and that the switch element (6, 6') closes an electric circuit (7, 7') between the two ends (8A, 8B) of the at least one coil (4A, 4B, 4C; 4A', 4B'), so that an electric current and an induced magnetic field that decelerates the rotational speed of the rotating part (2, 2') and of the tool connectable thereto is induced in the coil (4A, 4B, 4C; 4A', 4B') of the electrodynamic transducer (3, 3').

## Revendications

1. Poignée médicale, notamment dentaire, fonctionnant au fluide (1, 1') et comportant une pièce rotative (2, 2') pouvant être mise en rotation par un fluide d'entraînement pour entraîner un outil pouvant être relié à la pièce rotative (2, 2') et un convertisseur électrodynamique (3, 3') entraîné par la pièce rotative (2, 2') pour l'induction d'une tension électrique, le convertisseur électrodynamique (3, 3') comprenant au moins une bobine (4A, 4B, 4C; 4A', 4B') et un élément magnétique (5, 26), **caractérisée par** au moins un élément de commutation (6, 6') pour l'ouverture et la fermeture au choix d'un circuit électrique (7, 7') entre les deux extrémités (8A, 8B) de l'au moins une bobine (4A, 4B, 4C; 4A', 4B') de manière à ce que, lorsque le circuit électrique (7, 7') est fermé, un courant électrique et un champ magnétique d'induction ralentissant la vitesse de rotation de la pièce rotative (2, 2') et de l'outil pouvant lui être relié puissent être induits dans la bobine (4A, 4B, 4C; 4A', 4B') du convertisseur électrodynamique (3, 3').

2. Poignée médicale, notamment dentaire, fonctionnant au fluide (1, 1') selon la revendication 1, **caractérisée en ce que**
l'élément magnétique (5, 26) est prévu au niveau de la pièce rotative (2, 2') de manière à ce que l'élément magnétique (5, 26) puisse être mis en rotation par la pièce rotative (2, 2').

3. Poignée médicale, notamment dentaire, fonctionnant au fluide (1, 1'), selon la revendication 1 ou 2, **caractérisée en ce que**
l'au moins une bobine (4A, 4B, 4C; 4A', 4B') est enroulée autour d'un noyau de bobine à coercitivité basse (9).

4. Poignée médicale, notamment dentaire, fonctionnant au fluide (1, 1'), selon la revendication 3, **caractérisée en ce que**
le noyau de bobine à coercitivité basse (9) consistant en une ou plusieurs couches de préférence isolées électriquement les unes des autres a une forme annulaire et entoure l'élément magnétique (5, 26) sur sa circonférence extérieure.

5. Poignée médicale, notamment dentaire, fonctionnant au fluide (1, 1'), selon une des revendications précédentes, **caractérisée en ce que**
le convertisseur électrodynamique (3, 3') comprend plusieurs, de préférence trois, bobines (4A, 4B, 4C; 4A', 4B') qui sont disposées de manière particulièrement préférentielle sensiblement régulièrement autour de l'élément magnétique (5, 26).

6. Poignée médicale, notamment dentaire, fonctionnant au fluide (1, 1'), selon une des revendications précédentes, **caractérisée en ce que**
l'élément de commutation (6, 6') comprend une ou plusieurs diodes semiconductrices, notamment des thyristors.

7. Poignée médicale, notamment dentaire, fonctionnant au fluide (1, 1'), selon la revendication 6, **caractérisée en ce que**
l'élément de commutation (6, 6') comprend deux diodes Zener disposées en anti-série ou une diode bidirectionnelle.

8. Poignée médicale, notamment dentaire, fonctionnant au fluide (1, 1'), selon la revendication 6, **caractérisée en ce que**
l'élément de commutation (6, 6') présente au moins une diode électroluminescente émettant un rayonnement électromagnétique et au moins un élément semiconducteur recevant le rayonnement.

9. Poignée médicale, notamment dentaire, fonctionnant au fluide (1, 1'), selon une des revendications précédentes, **caractérisée en ce que**
l'élément de commutation (6, 6') comprend au moins une résistance électrique, notamment une résistance liée à la tension ou une résistance liée à la température.

10. Poignée médicale, notamment dentaire, fonctionnant au fluide (1, 1'), selon une des revendications précédentes, **caractérisée en ce que**
le convertisseur électrodynamique (3, 3') comprend au moins une bobine de commande (10) à laquelle un consommateur électrique (11) est raccordé ou qui est reliée opérationnellement à un capteur.

11. Poignée médicale, notamment dentaire, fonctionnant au fluide (1, 1'), selon une des revendications précédentes, **caractérisée en ce que**
l'élément de commutation (6, 6') comprend un circuit de commutation électronique, notamment un microcontrôleur.

12. Poignée médicale, notamment dentaire, fonctionnant au fluide (1, 1'), selon une des revendications précédentes, **caractérisée en ce que**
l'élément de commutation (6, 6') limite la vitesse de rotation de la pièce rotative (2, 2') et de l'outil qui peut lui être relié à une valeur d'environ 300.000 à 150.000 rotations par minute, de préférence à une valeur de l'ordre de 275.000 à 200.000 rotations par minute, de manière particulièrement préférentielle à environ 250.000 rotations par minute.

13. Poignée médicale, notamment dentaire, fonctionnant au fluide (1, 1'), selon une des revendications précédentes, **caractérisée en ce que**
le convertisseur électrodynamique (3, 3') et l'ensemble de l'élément de commutation (6, 6') sont reçus dans la poignée (1, 1').

14. Poignée médicale, notamment dentaire, fonctionnant au fluide (1, 1'), selon une des revendications précédentes, **caractérisée en ce que**
il est prévu plusieurs bobines (4A, 4B, 4C; 4A', 4B'), chaque bobine (4A, 4B, 4C; 4A', 4B') étant reliée à un élément de commutation (6, 6') et les éléments de commutation (6, 6') présentant différentes valeurs de commutation afin de pouvoir induire les champs magnétiques d'induction de manière séquentielle.

15. Procédé de limitation de la vitesse de rotation médicale, notamment dentaire, fonctionnant au fluide (1, 1'), selon une des revendications précédentes, **caractérisé en ce que**
une tension est induite par la mise en rotation de la pièce rotative (2, 2') dans l'au moins une bobine (4A, 4B, 4C; 4A', 4B') du convertisseur électrodynamique (3, 3') et que l'élément de commutation (6, 6') ferme un circuit électrique (7, 7') entre les deux extrémités (8A, 8B) de l'au moins une bobine (4A, 4B, 4C; 4A', 4B') de manière à ce que, dans la bobine (4A, 4B, 4C; 4A', 4B') du convertisseur électrodynamique (3, 3'), soient induits (2, 2') du courant électrique et un champ magnétique d'induction ralentissant la vitesse de rotation de la pièce rotative (2, 2') et de l'outil pouvant lui être relié.
